# EUROPEAN PATENT SPECIFICATION

(11) **EP 0 984 809 B1**
(45) Date of publication and mention of the grant of the patent: **03.08.2005**
(21) Application number: 98922541.2
(22) Date of filing: 15.05.1998
(51) Int. Cl.: A61M 25/06, A61B 17/34, A61M 5/42, A61M 25/02

(54) **APPARATUS FOR THE INTRAOSSEOUS INTRODUCTION OF A DEVICE SUCH AS AN INFUSION TUBE**
EINRICHTUNG ZUM INTRAOSSALEN EINSETZEN EINES GERÄTS, SOWIE EIN INFUSIONSSCHLAUCH
APPAREIL PERMETTANT L'INTRODUCTION INTRA-OSSEUSE D'UN DISPOSITIF TEL QU'UNE TUBULURE A PERFUSION

(30) Priority: 16.05.1997 CA 2205623
(43) Date of publication of application: 15.03.2000
(73) Proprietor: Pyng Medical Corporation, British Columbia V6H 3Z6 (CA)
(72) Inventor: FINDLAY, Judith, M., Richmond, British Columbia V7E 3Z9 (CA); JOHNSON, David, L., Burnaby, British Columbia V5J 2N6 (CA); KRASNICH, Misha, Richmond, British Columbia V6Y 1N4 (CA); JACOBS, Michael, W., Surrey, British Columbia V3X 2S4 (CA)
(74) Representative: Nash, David Allan
(86) International application number: PCT/CA1998/000492
(87) International publication number: WO 1998/052638

(56) References cited:
- US-A- 2 448 708
- US-A- 3 791 386
- US-A- 3 815 605
- US-A- 4 659 329
- US-A- 4 874 380
- US-A- 5 364 361
- US-A- 5 520 650

## Description

### FIELD

The present invention relates to an apparatus for infusing liquids into and to the aspiration of the bone marrow from humans and animals. In particular, the present invention pertains to infusion and aspiration of the bone marrow under emergency and field conditions.

### BACKGROUND OF THE INVENTION

Usually drugs and liquids are delivered to patients through a catheter or intravenously in a peripheral blood vessel. This method is satisfactory in cases where the blood pressure of the patient is at normal levels. However, when blood pressure drops, for example, during a heart attack, drug overdose, or severe hemorrhaging, the peripheral blood vessels collapse and access to these vessels is difficult or impossible. In such cases, an alternative to intravenous infusion is intraosseous infusion. An intraosseous infusion apparatus may be used to infuse drugs and other liquids into the bone marrow under such emergency conditions. In particular, an intraosseous device is used to penetrate the patient's skin, the subcutaneous layer between the skin and the top of the cortical layer of the bone, the cortical layer of the bone, and the bone marrow, and to supply drugs or fluids directly to the blood supply system of the bone. Typically, the sternum, femur, tibia or other bone near the skin is used. Intraosseous infusion can also be used on patients with blood vessels that are hard to find and on young children whose blood vessels are small and also hard to find. Intraosseous infusion can also be used in emergency or battlefield conditions where quick intravascular access may make the difference between life and death. The caregivers in these situations have low levels of training and need an intraosseous device that is simple and rapid to use.

Although intraosseous infusion is a feasible alternative to intravascular infusion, it has not met with widespread acceptance and popularity for a variety of reasons. One reason for this is the practical difficulty in inserting the infusion needle to the proper depth in the bone in order to access the marrow. One method to overcome this problem has been to use a stop or marker on the needle to indicate when the needle has penetrated to a particular depth. This method has not been effective since it requires an estimation of the required depth and careful control during advancement of the needle. Skin and tissue thickness overlying the bone range from 3 mm to 30 mm and thus the skin surface cannot be used as a reliable reference point. A trained individual like a doctor would be needed to determine the correct depth and insert the intraosseous device. This can be difficult even for highly skilled professionals. Another method to overcome this problem has been to monitor the resistance to the penetration of the infusion needle. The resistance is high when the needle goes through the cortical layer of the bone but decreases when it hits the bone marrow. This method is not very effective since resistances may vary. Again, a highly trained individual is required to advance the intraosseous needle or tube slowly and feel for the changes in resistance.

Intraosseous penetration of the cortical layer of the bone to the bone marrow is also needed when a sample of bone marrow from a patient must be taken. Again a needle or tube must be inserted through the subcutaneous layer into the bone so that the bone marrow can then be aspirated. Again, only a highly trained individual can accurately determine the depth of the penetration of the tube or needle into the bone marrow.

In U.K. Pat.No. 1,315,796, issued to Pashenichny et al., a device for intraosseous injection is disclosed consisting of an outer tube with a screw and a male thread on one end and an inner tube fitted into the outer tube. The device is drilled into the osseous tissue, the inner tube is removed and a cannula is connected to the outer tube. U.S. Pat.No. 4,969,870, issued to Kramer et al., discloses an apparatus for intraosseous infusions having a base positioned with its lower surface against the patient's skin and the infusion tube is pushed through the skin and then rotated to thread through the bone until continued rotation of the tube no longer advances the tube. In both of these devices, there is no automatic depth sensing mechanism. In U.S. Pat. No. 3,815,605, issued to Schmidt et al., an intraosseous device has pins or legs similar to a bone probe that penetrates through the subcutaneous layer. The user releases a compressed spring that exerts a force on and delivers energy to an infusion tube to cause it to penetrate the bone. A striker-pin holder that couples the spring to the infusion tube, engages a shoulder, which houses the bone probe, thereby impeding the penetration of the infusion tube into the bone. Although this device does have a bone probe which allows the bone cortical layer to be used as a reference point in determining the depth of the penetration of the infusion tube instead of the skin, there is no automatic release mechanism to prevent overpenetration of the bone marrow. Furthermore, when the striker-pin holder engages the shoulder, the excess energy released from the spring may drive the bone probe downwardly and over-penetrate the bone.

U.S. Patent No. 5,520,650 issued to Zadini discloses a device for inserting a cannula into a body cavity. A piston is pushed by hand so that an attached cannula penetrates the skin. Once well under the skin the operator releases the piston and the piston is urged to return by a spring bias creating a vacuum in the piston chamber. The vacuum draws body fluid into the cannula and piston chamber until the vacuum drops. With the vacuum low enough, the piston moves back against an arrest pin releasing an interface member to be urged forward by a spring causing an arrest rod to be locked which, in turn blocks further movement of the catheter or needle. Zadini requires a hollow cannula be inserted since it senses pressure in order to hold the cannula from causing the chain of events that locks an arrest rod and prevents further insertion of the hollow cannula. The needle tip is expected to encounter a fluid which will flow into the needle, destroying the vacuum in the chamber and allow the piston to move, thus triggering the arrest mechanisms of the device. Zadini also requires that the overlying tissue have sealing qualities. Thirdly, Zadini arrests movement of the cannula immediately upon entry into a suitable body cavity and does not detect relative position within a target cavity, but merely whether the cannula is in the cavity or not.

U.S. Patent No. 4,874,380 issued to Hesketh discloses a releasable catheter retaining device mounted on a patch which has a post to which is anchored a cable tie. The cable tie is used to engage a catheter. The sole function of the patch is to retain a catheter.

Battenfield (US-A-5 364 361) discloses a template for instructing proper insertion of a means for draining a distended bursa. The template is for use on either a right or left knee and has locating indicia marked on it for visual alignment with the patella and tibia. Since visual alignment alone is unreliable it would be desirable to combine such a method with a more mechanical method of alignment.

Other similar apparatus for intravascular infusion (U.S. Pat. No. 5,527,290 issued to Zadini et al. and U.S. Pat. No. 5,480,388 issued to Zadini et al.) and tracheotomies (U.S. Pat. No. 4,556,059 issued to Adamson, Jr.) may have automatic trigger mechanisms that use a spring for self-propelled insertion. None of the prior art discloses a release mechanism that controls the depth of penetration of the penetrating means inserted at arbitrary speed through arbitrary thickness of overlying tissue, against an unknown resistance.

Another problem in employing intraosseous infusion is the need for quickly and easily finding the proper location on a patient's body for insertion of the infusion tube. A semi-skilled caregiver in an emergency situation would not be able to quickly identify the target location for intraosseous infusion. Prior art discloses templates for guiding the insertion of syringes for draining the bursa of the knee and for insertion of spinal marker needles. A template for guiding a caregiver to the correct location for draining the bursa of the knee along with the hypodermic needle used in the process is disclosed in U.S. Pat.No.5,364,361, issued to Battenfield. U.S. Pat.No. 4,985,019, issued to Michelson, teaches a X-ray marker disc with a grid pattern and indicia for determining the location and orientation of the spinal marker needle. There is a need for a template to guide the placement of an intraosseous infusion apparatus so that a semi-skilled caregiver can accurately and very quickly determine the site of intraosseous infusion.

A third problem with intraosseous infusion is that strain and stress on the infusion tube that protrudes above the skin may cause dislodgment of the tube from the bone, tearing of the skin or overpenetration of the infusion tube. One cause of such stress is the movement of skin and tissue which may cause strain on the infusion tube and may dislodge it. The infusion tube may be placed under tension by the intravenous fluid supply tube. Forces or pressures from objects pressing on the intraosseous infusion site may push the infusion tube too far into or through the bone. This problem is particularly difficult when a patient is being transported in an ambulance or in a war zone where movement of the patient under uncontrolled conditions is required.

Prior art discloses several devices for supporting catheter tubing, for example U.S. Pat.No. 4,397,641, issued to Jacobs, which teaches a catheter support member and U.S. Pat.No. 5,456,671, issued to Bierman, which teaches a catheter anchoring system. Prior art also discloses several protective coverings for the catheter infusion sites as in U.S. Pat.No. 5,074,847, issued to Greenwell et al., which discloses a shielding device and a method for holding a heparin lock secured to a catheter and U.S. Pat.No 5,449,349, issued to Sallee et al., which discloses an intravenous tubing cover/protector. These supports are customized for catheters. Thus, a need for an intraosseous tube support which can create a protective loop of slack, and a protector covering the intraosseous infusion site and intraosseous infusion tube exists.

It should therefore be appreciated that there is a significant need for an intraosseous infusion or aspiration apparatus and a related method that can be used quickly and easily by even low-skilled caregivers in emergency or field conditions. Further there is needed such a device that provides for quick positioning of the target area and one that enables semi-skilled users to reliably and accurately position an intraosseous infusion device. There is also a need for such a device that provides relief from the stress and strain placed on the tubing and protection against dislodgment or overpenetration.

### SUMMARY OF THE INVENTION

According to the invention, there is provided an apparatus for intraosseous fluid infusion and aspiration of bone marrow beneath a bone cortical layer of a patient. The apparatus has an operative end that refers to the bone penetrating end of the apparatus and a remote end opposite to the operative end. The apparatus has an introducer, comprising a housing assembly, a spring assembly a bone probe assembly, a release mechanism, an infusion tube and a coupler coupling the introducer to the infusion tube. The infusion tube may have a bone portal and a hollow flexible tube affixed to the bone portal. The infusion tube infuses fluid to and aspirates tissue from the bone marrow. The release mechanism removes substantially all of the force exerted on che infusion cube once the bone portal has penetrated the bone marrow a predetermined distance.

The apparatus also has a spring assembly with a spring that is compressed between the remote end of the inner sleeve and the remote end of the bone probe assembly, and functions to hold these two parts in a relative initial position.

The bone probe assembly is slidable into the housing assembly. As a user exerts force onto the housing assembly, the spring compresses and the bone portal penetrates the bone cortical layer. When the housing is withdrawn, the infusion tube is left in the body of the patient with the bone portal embedded in the bone marrow and the hollow infusion tube expending out of the skin.

The housing is, further, comprised of a cylindrical outer sleeve with a ball race in an interior surface at the operative end of the sleeve and a cylindrical inner sleeve which is slidably insertable in the outer sleeve. The inner sleeve has a plurality of ball holes circumferentially spaced in the operative end of the sleeve such that the inner and outer sleeve can be coupled through a plurality of bails located partly in these ball holes and partly in the ball race of the outer sleeve.

Specifically, the bone probe assembly is removably insertable in the inner sleeve. A port ion of the outer surface of the bone probe assembly is conical in shape, decreasing in diameter towards the operative end of the infusion apparatus. When a user applies a force to the outer sleeve, the balls couple the outer sleeve to the inner sleeve, which couples the introducer to the infusion tube through a long slender sylet coupled to the remote end of the inner sleeve and over which the hollow infusion tube is mounted. As the user applies force to the outer sleeve, the entire apparatus moves towards the patient and the needles of the bone probe penetrate the skin and subcutaneous layers until they come to rest on the cortical layer. As the user continues to apply force to the outer sleeve, all parts of the apparatus, except for the bone probe assembly, continue to move toward the patient and the bone portal begins to penetrate the cortical layer. Because the bone probe assembly is in contact with the cortical layer and is slidable in the inner sleeve, the bone probe assembly does not move toward the patient. As more relative motion occurs between the bone probe assembly and the rest of the apparatus, the balls start to move down the conical outer surface of the bone probe assembly, and thus move radially inward. When the infusion tube has penetrated the correct distance into the cortical layer, the balls have moved inward until they no longer couple the outer sleeve to the inner sleeve. This action is the release mechanism which releases the outer sleeve from the rest of the apparatus. At this point, any downward force exerted by the user to the outer sleeve, is not transferred to the infusion tube, thereby preventing any further penetration of the infusion tube into the bone.

The bone probe assembly is also coupled to the inner sleeve through pins that engage pin slots in the inner sleeve. The pin slots allow a displacement of the bone probe assembly relative to the inner sleeve that is slightly beyond the displacement at which the release mechanism is activated. The pins are located in pin holes in the annular band of the bone probe assembly adjacent to the remote end of the conical surface down which the balls travel as the release mechanism is activated. A bone probe ring is adjacent to the operative end of the conical surface. From the bone probe ring, a plurality of needles project out in a circle.

Furthermore, the bone probe assembly has an axial opening. In the axial opening there may be an infusion tube surrounded by two support sleeves and a stylet removably attached to the inner sleeve and passing through the infusion tube and contacting a bone portal. The support sleeves brace the infusion tube when a force is applied to the outer sleeve.

In another aspect of the invention, a release mechanism is provided. This release mechanism is designed to control the distance over which a user exerted force can act. The displacement of the bone portal relative to the bone is always identical regardless of the speed at which the force is exerted by a user, regardless of whether the force exerted by a user is constant or variable, and regardless of the magnitude of the force exerted on the bone portal. This is in contrast to a spring trigger mechanism where the apparatus is propelled forward by energy stored in a spring bat the distance propelled cannot be accurately controlled because all the energy stored in the spring must be delivered to the bone and the user.

In another aspect of the invention, the intraosseous infusion and aspiration apparatus may be optimized for infusion and aspiration of different bones with different bone resistances, different overlying skin and subcutaneous resistances, and different depth of penetrations by modifying several variables. The spring constant, the attributes of the bone probe needles, the axial displacement of the balls, the angle of the conical surface on the bone probe assembly, the angle of the ball contacting surface on the outer sleeve and the size of the pin slots may be adjusted to yield different bone penetration depths, different maximum penetration depths, different applied force, and different maximum applied force that would be needed for different bones.

Accordingly, the present invention is embodied in an intraosseous infusion and aspiration apparatus and related method which effectively allows a user to place an infusion tube in the bone marrow of the patient without having to estimate the penetration depth or bone's resistance to penetration and without having to estimate the target area of the placement of the infusion tube. Essentially, the present invention provides an object to be positioned, a pusher to push on, a coupler to couple the pusher to the object being positioned, a position probe that senses the location of the object to be positioned relative to a reference point and a release mechanism that removes substantially all of the force applied to the object, once the object is correctly positioned relative to the reference point. More specifically, the object being positioned is the infusion tube. The pusher corresponds to the outer pusher sleeve and the coupler to the balls. The position probe corresponds to the bone probe assembly.

There may be additionally provided an elongated remover in the shape of a rod that has threads at one end. After an infusion is complete, the remover is inserted into the infusion tube so that it engages the threads in the bone portal. A force is applied to the remover in the direction away from the bone thereby extracting the bone portal from the bone.

### BRIEF DESCRIPTION OF THE DRAWINGS

The novel features believed to characterize the invention are set forth in the appended claims. The invention, itself, however, as well as other features and advantages thereof, will be best understood by reference to the detailed description which follows, read in conjunction with the accompanying drawings, wherein:
Fig. **1** is an exploded isometric view of che intraosseous infusion and aspiration apparatus;
Fig. **2A** is a perspective view of an infusion tube with the hollow flexible tube, tube connector and bone portal;
Fig. **2B** is a perspective view of the bone portal and a portion of the attached hollow tube;
Fig. **2C** is a perspective view of the conically tapered bone portal and a portion of the attached hollow tube.
Fig. **3A** is a side elevation view of the bone portal:
Fig. **3B** is a sectional view of Fig. 3A;
Fig. **3C** is a sectional view of the conically tapered bone portal.
Fig. **4** is a cut-away perspective view of the assembled intraosseous infusion and aspiration apparatus;
Fig. **5** is a sectional view of the intraosseous infusion and aspiration apparatus;
Fig. **6** shows the axial and radial displacement of the ball during release;
Fig. **7** shows the angles on the ball race and the tapered surface of the bone probe assembly in an example of a release mechanism of an intraosseous infusion and aspiration apparatus;
Fig. **8** shows the forces acting on the ball, inner sleeve, and outer sleeve during an initial phase of the release mechanism;
Fig. **9** shows the forces acting on the ball, inner sleeve, and outer sleeve during a late phase of the release mechanism;
Fig. **10** is the first stage in the use of the intraosseous infusion and aspiration apparatus showing the bone probe against the bone cortical layer and the bone portal just beginning to penetrate the skin;
Fig. **11** is the second stage in the use of the intraosseous infusion and aspiration apparatus showing that the bone portal has penetrated the bone cortical layer;
Fig. **12** is the third stage in the use of the intraosseous infusion and aspiration apparatus showing that the inner sleeve has been released from the outer sleeve leaving the bone portal at the correct depth in the bone marrow;
Fig. **13** is the fourth stage in the use of the intraosseous infusion and aspiration apparatus showing that the apparatus has disengaged from the skin of the patient and the infusion tube has been left in the patient;
Fig. **14** shows the placement of the template patch on the skin over the patient's sternal bone;
Fig. **15** shows the template patch;
Fig. **16** shows the placement of the intraosseous infusion and aspiration apparatus at the target zone of the template patch;
Fig. **17** shows the template patch after the intraosseous infusion and aspiration apparatus has inserted the bone portal into the bone marrow and has been disengaged, and the infusion tube has been connected to the connector tube;
Fig. **18** shows the covering for the template patch; and
Fig. **19** shows an example of a target patch designed for use at the tibial site for intraosseous infusion.

### DETAILED DESCRIPTION WITH REFERENCE TO DRAWINGS

In the following description it is to be understood that the apparatus has two ends: an operative end that refers to the bone penetrating end of the apparatus and the opposite end referred to as the remote end.

The intraosseous infusion and aspiration apparatus **1** serves as an introducer that introduces an object, an infusion tube **17,** to a specific position and a predetermined depth in the bone marrow **55.** The introducer is comprised of an outer sleeve **5,** a bone probe assembly **3,** and a coupler **7** that couples the infusion tube **17** to the outer sleeve **5.** Thus the infusion tube **17** is positioned in the bone marrow **55** through the action of an outer sleeve **5,** a bone probe assembly **3** that senses the location of the infusion tube **17** that is being positioned, and a coupler **7** that couples the outer sleeve **5** to the infusion tube **17** so that force exerted on the outer sleeve **5** is transferred to the infusion tube **17.** The automatic release mechanism of the apparatus involves all parts except for the infusion tube **17.**

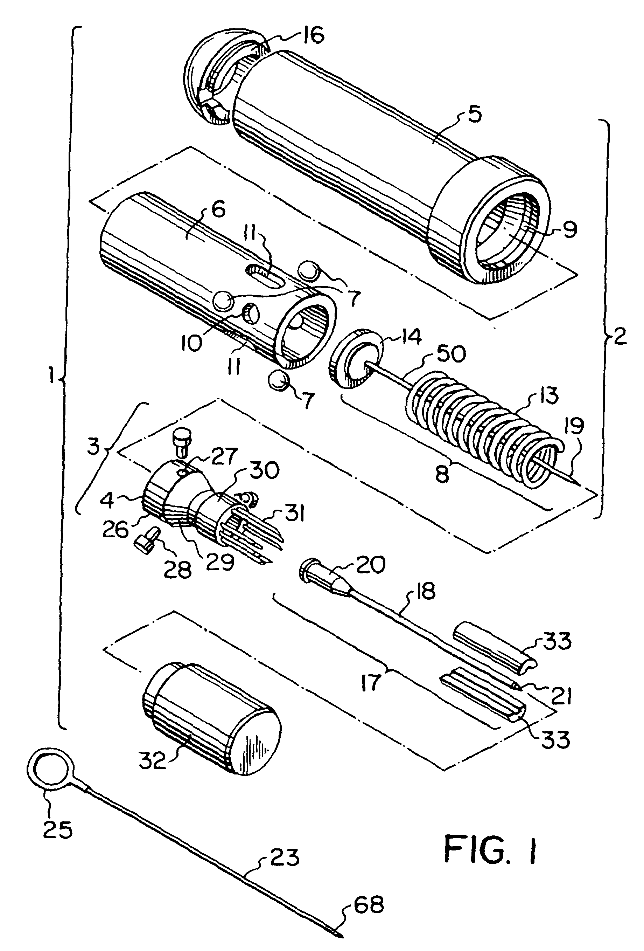

A cross-section of the intraosseous infusion and aspiration apparatus **1** is shown in its preferred embodiment in Fig. **1.** The apparatus has a housing assembly **2,** a plurality of balls **7,** a spring assembly **8,** a stylet **50,** stylet mount **48,** stylet base **14** and a bone probe assembly **3.**

The housing assembly **2** has an outer sleeve **5** and an inner sleeve **6.** The hollow outer sleeve **5** is cylindrical in shape and serves as the surface to which force is applied. A ball race **9** is formed in the interior wall of the operative end of the hollow outer sleeve **5.** The hollow outer sleeve **5** also has a cap **16** with a projection or thread that allows it to fit snugly into the remote end of outer sleeve **5.**

The inner sleeve **6,** also of cylindrical shape and hollow, slidably fits inside the hollow outer sleeve **5**. The inner sleeve **6** has a plurality of ball holes **10** circular in shape and a plurality of elongated pin slots **11** circumferentially spaced about the operative end of the inner sleeve **6.**

A plurality of balls **7** serve as the coupler coupling the outer sleeve **5** to the infusion tube **17**. The balls **7** couple the outer sleeve **5,** to the inner sleeve **6** which is releasably attached to the infusion tube **17**. The balls **7** are of a diameter slightly smaller than the ball holes **10** and fit partly in the ball holes **10** of the inner sleeve **6** and partly in the ball race **9** of the hollow outer sleeve **5** coupling the hollow outer sleeve **5** with the inner sleeve **6.**

The spring assembly **8** has a helical spring **13** which is positioned inside the inner sleeve **6,** abutting a stylet base **14.** One side of the stylet base **14** abuts a retaining lip **15** of the interior of the inner sleeve **6** proximate a remote end thereof. On the opposite side, the stylet base **14** has a projection that fits snugly into the remote end of the spring **13**. The stylet base **14** couples the compression forces from spring **13** to the inner sleeve **6.**

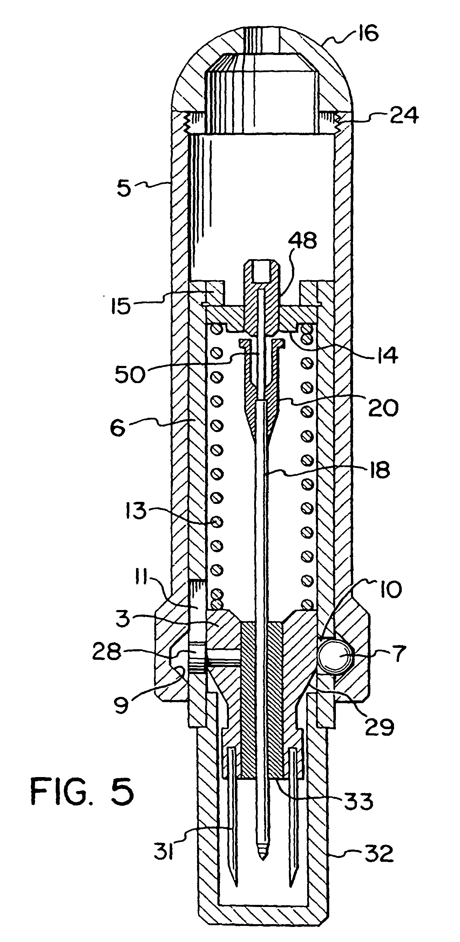

A stylet **50** is connected to a stylet mount **48** (see Fig. **5**) affixed to the center of the stylet base **14**. Stylet base **14** is coupled to the inner sleeve **6** and the spring **13.** The force exerted onto the hollow outer sleeve **5** is transferred to the inner sleeve **6** through the balls **7** coupling the two bodies **5, 6** together and is further transferred to the spring **13** and stylet **50** through the retaining lip **15** and stylet base **14.** The stylet **50** is rigid and is inserted into the infusion tube **17** to push the infusion tube **17** into the bone **40.**

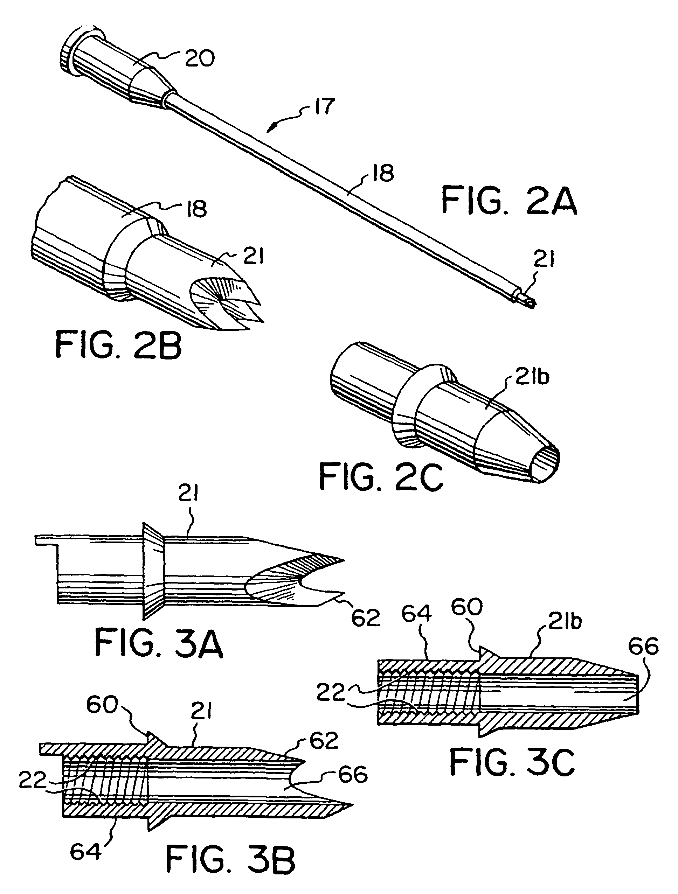

The infusion tube **17** consists of flexible tubing **18,** and a bone portal **21.** The flexible tubing **18** is a hollow, elongated, flexible tube connected to a tube connector **20** (Fig. **2A**) at the remote end. Referring to Fig. **2A** and **2B,** the flexible tubing **18** is connected to the bone portal **21.** The flexible tubing **18** is attached to the bone portal **21** providing a fluid passageway from the flexible tube **18** to the bone portal **21.** Referring to Figures **2A, 2B, 2C, 3A, 3B** and **3C,** the bone portal **21** is made of a rigid material such as stainless steel and has a bore **66** which communicates with an opening at its operative end to allow the infusion of fluid into the bone marrow. On the interior surface of the bore **66** are threads **22.** An annular shoulder **60** serves as a stop for the hollow, flexible tubing **18** that is attached to the exterior surface of the bone portal **21.** The end of the bone portal **21** is beveled to form sharp points **62.** Alternatively, as seen in Figures **2C** and **3C,** the bone portal **21b** may be conically tapered.

As seen in Fig. 1, the remover **23** has a slender rod threaded at its end with threads **68** dimensioned to register with the threads **22** on the interior bore of the bone portal **21** and a handle **25** at the remote end is used to remove the flexible tubing **18** and bone portal **21** from the bone marrow after the infusion is complete.

Referring to Fig. **1,** the intraosseous infusion and aspiration apparatus **1** further includes a bone probe assembly **3** that serves as a position probe allowing the location of the object, the infusion tube **17,** to be positioned relative to a reference point. The bone probe assembly **3** comprises a bone probe ring **4,** a plurality of pins **28,** and a plurality of needles **31.** The bone probe ring **4** comprises an annular band **26** that is dimensioned to slide into one end of the inner sleeve **6.** This annular band **26** of the bone probe ring **4** has a plurality of pin holes **27.** A plurality of pins **28** can be put through these pin holes **27** and into the elongated pin slots **11** in the inner sleeve **6** further slidably securing the bone probe assembly **3** to the inner sleeve **6.** The bone probe ring **4** also has a conical surface **29** (a ramp in transverse drawings) adjacent to the annular band **26.** A ring of needles **31** protrude out from the operative end **30** of bone probe ring **4.** There is a protective covering **32** that covers the needles **31** to protect an administrator from accidental contact with the needles **31.**

The bone probe needles **31** serve as a reference for the measurement of the distance through the bone that the bone portal **21** has penetrated since the needles **31** penetrate the skin and subcutaneous layers overlying the bone, but do not penetrate the bone.

The intraosseous infusion and aspiration apparatus **1** further includes longitudinally split support sleeves **33** located in the bore of the bone probe ring **4.** Support sleeves **33** brace the stylet **50** so that it does not buckle under the force applied to it to penetrate the bone.

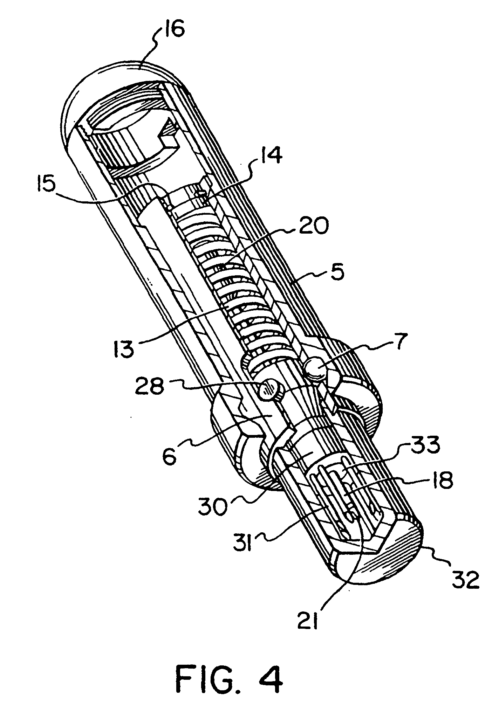

Referring to Fig. **4,** the assembled intraosseous infusion and aspiration apparatus **1** is shown in its position before use with a protective covering **32** over the bone probe needles **31.**

The intraosseous infusion and aspiration apparatus **1** can be optimized for infusion of different bones such as the sternum, the proximal and distal ends of the tibia, the femur, and the clavicle. These bones have different resistances to penetration thus the amount of force needed to insert the apparatus in the bone marrow of the bones may differ. Also, since different depths of penetration of bone to reach the bone marrow may be needed for different bones, the bone penetration distance of the bone portal may need to be adjusted. In addition, the skin and subcutaneous layers overlying the different bones may differ in thickness and their resistance to penetration. The bone probe ring **4** and spring **13** may have to be adjusted to compensate for these changes in the thickness and resistance of the skin and underlying tissue. The intraosseous infusion and aspiration apparatus **1** may also be customized for pediatric patients who usually have smaller bones with lesser resistance to penetration.

One feature of the intraosseous infusion and aspiration apparatus **1** that can be adjusted is the spring force applied to the bone probe ring **4.** The tips of the bone probe needles **31** serve as a reference point to determine the depth of penetration of the bone portal **21** through the cortical bone layer **40** and bone marrow **55.** The magnitude of the spring force needed to force the bone probe needles **31** to penetrate the skin **56** and subcutaneous layer **57** so that it abuts the bone cortical layer **40** is dependent on the bone probe needle **31** configuration, the type of tips of needles **31,** the size and the number of needles **31,** and the resistance of the skin **56** and subcutaneous layer **57.** For example, if the number of needles is decreased then a weaker spring force may be used for the bone probe needles **31** to penetrate the same skin and underlying tissue. Since different anatomical sites have different resistances in the skin and underlying tissues, the spring force and the bone probe needles **31** can be adjusted to obtain optimum characteristics for the penetration of the bone probe needles **31** to the cortical bone **40.**

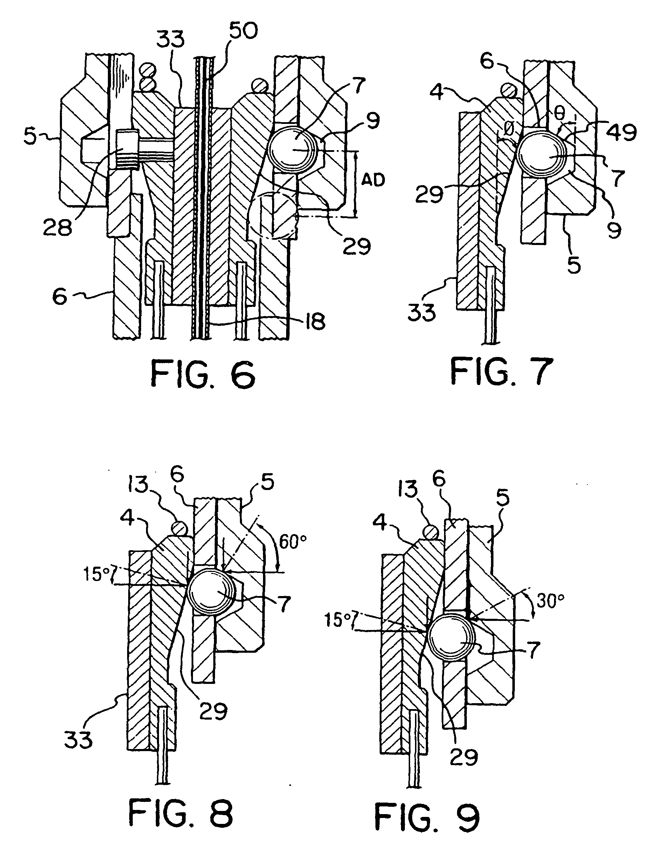

Another feature of the apparatus **1** that can be adapted is the release mechanism. Referring to Fig. **5,** the ball release mechanism comprises a plurality of balls **7,** the ball race **9,** the ball holes **10,** the spring assembly **8** and the conical surface **29** of the bone probe ring **4** and the bone probe assembly **3** itself. Referring to Fig. **6,** the starting position of the ball **7** before release is on the remote end and the ending position of the ball **7** is proximate the operative end of the conical surface **29** of the bone probe ring **4.**

Referring to Fig. **7,** the angle of the conical surface **29** on the bone probe ring **4,** the angle of the ball contacting surface **49** of the ball race **9** and the spring force can be adjusted to determine the maximum bone portal penetration force available to insert the infusion tube **17** to a predetermined depth. For example, if the angle of the conical surface with the axis of the bone probe assembly φ (see Fig. 7) is increased for a constant ball race contacting surface angle θ and constant spring constant, the maximum available bone portal penetration force will decrease. If the angle θ is increased as angle φ and the spring constant are kept constant, the maximum available bone portal penetration force will increase. If the spring constant is increased for constant angle θ and angle φ, the maximum available bone portal force will increase. If this maximum bone portal force is exceeded, the apparatus **1** is released without damage. Since this force is much less than the force at which mechanical failure occurs, the apparatus will not be damaged and the patient will not be injured.

Fig. 8 shows the forces on the ball release mechanism in an initial phase where the ball is positioned at the remote end of the conical surface **29** of the bone probe ring **4** . In this initial phase, forces up to the maximum force may be applied without premature release. Fig. **9** shows the forces on the ball release mechanism as the balls are positioned in the operative end of the conical surface **29** of the bone probe ring **4.** In this late phase, the apparatus **1** may release prematurely since there is a greater horizontal force acting on the ball **7** forcing the ball **7** onto the ramp **29.** The horizontal force tends to push the bone probe up and causes release. In this phase, the axial displacement of the bone assembly relative to the inner sleeve **6** is determined by the angle φ, the angle θ and the diameter of the balls **7.** Changing one of these design variables will change the axial displacement that occurs in this phase.

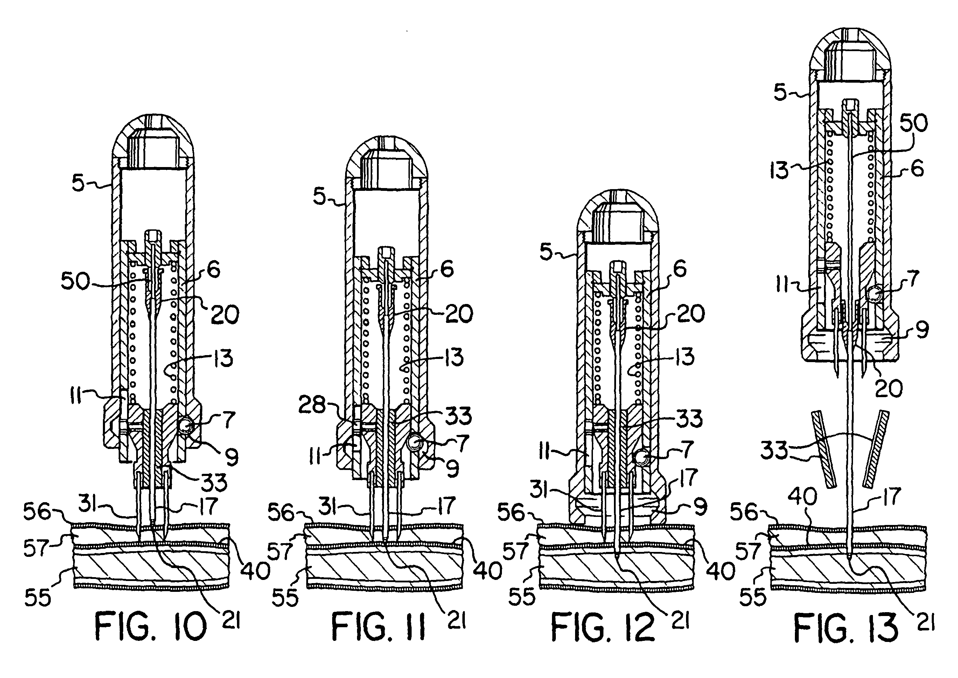

Another aspect of the intraosseous infusion and aspiration apparatus **1** that can be adjusted is the size of elongated pin slots **11** (see Fig. **10-13)** in the inner sleeve **6** proximate the operative end. These pin slots **11** determine the maximum axial displacement of the bone probe assembly **3** in relation to the inner sleeve **6.** Because the infusion tube **17** is coupled to the inner sleeve **6,** these pin slots **11** also determine the maximum penetration depth of the infusion tube **17** in relation to the bone probe needles **31.** Thus, if there is a failure in the release mechanism, this feature ensures that the bone portal **21** does not overpenetrate the bone marrow and cause injury to the patient.

The ball race **9** allows for rotational decoupling between the hollow outer sleeve **5** and the inner sleeve **6.** Optionally, ball race sections could be provided in order to provide limited decoupling between the hollow outer sleeve **5** and the inner sleeve **6.** With such a feature, if the needles **31** were decoupled by use of a bearing on the bone probe assembly for example, it would be possible to apply torque to the bone portal **21** in order to assist its penetration into the bone. Alternatively, other moethods may be used to couple the outer sleeve **5** to the inner sleeve **6,** such as a pin in outer sleeve **5,** engaging a slot of inner sleeve **6.**

The operation of the intraosseous infusion and aspiration apparatus **1** and its release mechanism is shown in Fig. **10, 11, 12, 13.** The apparatus **1** contains a release mechanism for disconnecting the infusion tube **17** and the bone portal **21** from the outer sleeve **5** when the bone portal **21** is at a specific depth relative to the outer surface of the cortical bone **40** thereby preventing the bone portal **21** from penetrating beyond the bone marrow **55** and out the opposite cortical layer of the bone. Specifically, the intraosseous infusion and aspiration apparatus **1** is placed on the target location perpendicular to the skin of the patient. A force is applied so that the bone probe needles **31** go through the skin **56.** A portion of the bone portal **21** also enters the subcutaneous layer **57** (see Fig. **10).**

Referring to Fig. **10,** the balls **7** are in the ball holes **10** and ball race **9.** The pins **28** sit at the operative end of the elongated pin slots **11.** As more force is applied on the hollow outer sleeve **5,** as seen in Fig. **11,** the outer sleeve **5** and the inner sleeve **6** move towards the operative end of the apparatus **1.** Since the infusion tube **17** is coupled through the stylet **50** to the stylet base **14** which is coupled to the inner sleeve **6** which in turn is coupled to the outer sleeve **5,** as force is exerted on outer sleeve **5,** the bone portal **21** penetrates the bone cortical layer **40.** Since the bone probe assembly **3** has not changed in position, there is relative movement of the pins **28** on the bone probe assembly **3** towards the remote end of the elongated pin slots **11** of the inner sleeve **6.** The balls **7** coupling the inner sleeve **6** to the hollow outer sleeve **5** are allowed to move out of the ball race **9** of the hollow outer sleeve **5** and through the ball holes **10** in the inner sleeve **6** toward the center of the apparatus since the hollow outer sleeve **5** is moving down relative to the bone probe assembly **3** and space is created into which the balls **7** can move.

As seen in Fig. **12,** as more force is applied, the penetration of the infusion tube **17** into the bone marrow **55** takes place. Eventually the balls **7** have travelled radially inward towards the centre of the apparatus sufficiently so that they no longer make contact with the ramp surface **49** of the ball race **9** in the outer sleeve **5.** When this occurs (see Fig. **12**) force is no longer coupled from the outer sleeve **5** to the inner sleeve **6.** At this point, the infusion tube **17** has been released from its coupling to the outer sleeve **5.** The infusion tube **17** has been inserted to the correct depth.

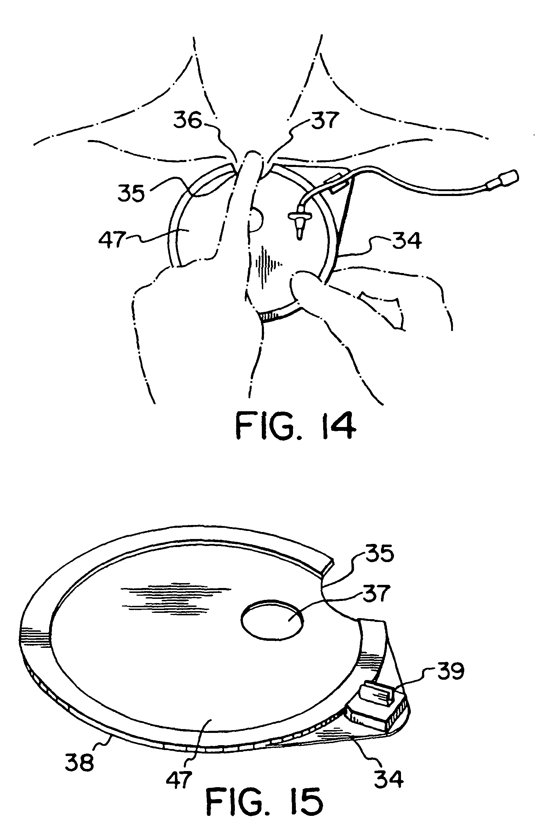

The hollow outer sleeve **5** has been pushed so that the operative end of the hollow outer sleeve **5** rests on the skin of the patient. The balls **7** have moved out of the ball race **9,** through the ball holes **10,** and down the conical surface **29** into the space between the bone probe assembly **3** and the inner sleeve **6,** uncoupling the hollow outer sleeve **5** from the inner sleeve **6.** Compressed spring **13** exerts a force on the bone probe assembly **3** against the stylet base **14** causing the balls **7** to be pressed outwardly against the outer sleeve **5,** thereby producing a frictional force between the outer sleeve **5,** the inner sleeve **6** and the bone probe assembly **3**. In Fig. **13**, the hollow outer sleeve **5** is pulled back, pulling the stylet **50** from the infusion tube **17.** The support sleeves **33** fall out as the apparatus **1** is removed. The infusion tube **17** can be connected to another tube **41** or directly to a source of drugs and fluid using the tube connector **20** on the infusion tube **17.**

This intraosseous infusion and aspiration apparatus **1** can be used in conjunction with a target/strain-relief patch **34** (Fig. **14).** The patch **34** is used as a guide to ensure that the intraosseous infusion and aspiration apparatus **1** is correctly positioned in the proper location on a bone. A prominent anatomical feature of the bone like a notch, a depression, or a bump is used as a reference point to determine the target location for the infusion or aspiration of the bone marrow of flat bones such as the sternum, or iliac crest and long bones such as the femur, the tibia, or the radius.

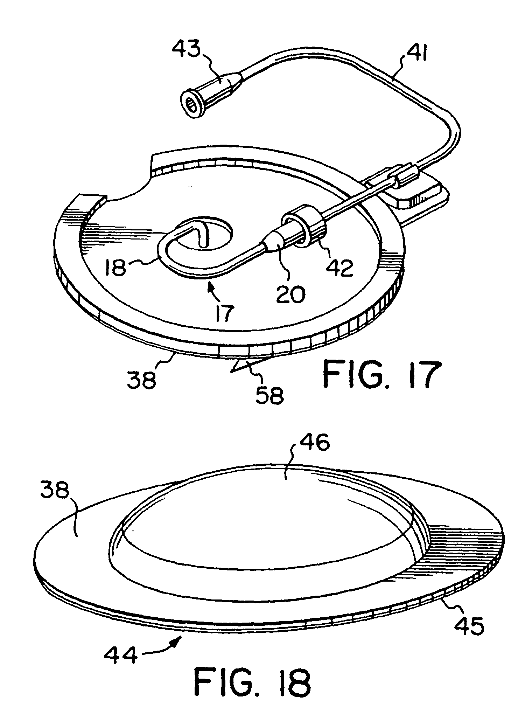

Referring to Fig. **14,** the patch **34** includes a patch base **47** which is used to locate a target zone **37** on the manubrium bone of a patient by placing the finger in peripheral notch **35** and at the same time locating the finger in the sternal notch **36** of the patient. Referring to Fig. **15,** a target zone **37** in the patch base **47** is positioned a predetermined distance away from the peripheral notch **35.** The target zone **37** is used to align the intraosseous infusion and aspiration apparatus **1** with a desired area of penetration of the patient. Also, the patch base **47** has an adhesive underside with a liner **58** that can be peeled to removably fasten the patch base **47** to the skin **56** of the patient. Liner **58** may be split such that it has two pieces that can be removed independent of one another. In addition, a fastening material **38** is present around the periphery of the patch base **47** so that a cover **44** may be placed on it and engage the fastening material **45** (Fig. **18).** The patch **34** also has a tube clamp **39** outside the fastening material **45** on an extension of the patch base **47.** The infusion tube **17** may be attached by the tube clamp **39** to the patch **34** and then connected to an intravenous tube through its tube connector **20.** In another embodiment, a connector tube **41** with a connector **42** and a connector **43** is attached to the tube clamp **39.** Connector tube **41** is attached to the tube connector **20** on the infusion tube **17** with connector **42,** and connector **43** is used to attach connector tube **41** to a source of fluids. The tube clamp **39** or the connector tube **41** decrease the strain on the bone portal **21** by creating the slack in the tube and also prevent the accidental dislodgment of the infusion tube **17** and the bone portal **21** by either clamping the infusion tube **17** or the connector tube **41** to the patch base **47.**

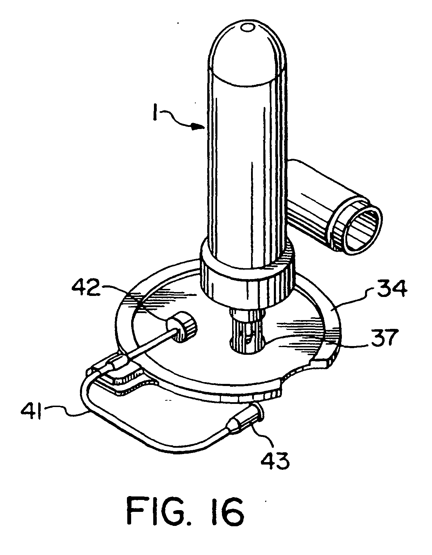

Referring to Fig. **16,** the intraosseous infusion and aspiration apparatus **1** is placed perpendicular to the patch **34** in the target zone **37.** After the bone portal **21** has been inserted into the bone marrow and the intraosseous infusion and aspiration apparatus **1** removed, the infusion tube **17** is connected to the connector tube **41** with the tube connector **20** and connector **42** as shown in Fig. **17**. Connector **43** of the connector tube **41** can be connected to a source of intravenous drugs or fluid.

Figure **18** shows a preferred embodiment of the cover **44** which has a dome **46** of transparent material with fastening material **45** around its periphery. The dome **46** can be placed on the patch base **47** and the fastening material **38** of the patch base **47** can engage with the fastening material **45** of the cover **44** to protect the site of infusion. The fastening materials can be hook and loop which allows the dome to be removed and reattached.

The intraosseous infusion and aspiration apparatus **1** can be used alone if a patch **34** is not available, or in conjunction with the patch **34.** The patch **34** may also be used with other intraosseous infusion and aspiration apparatus . When a patch **34** is used in conjunction with the intraosseous infusion apparatus **1,** the top half of the backing of the patch **34** is first removed to expose the adhesive lining on the underside. An appropriate anatomical marker on the appropriate bone is located, for example the sternal notch **36** in the manubrium bone of the patient. An index finger is placed on the anatomical marker perpendicular to the surface of the bone and the peripheral notch **35** on the patch **34** is arranged around the finger in the proper orientation. In this example, the peripheral notch **35** and the target zone **37** are over the patient's midline on the chest. The top half of the patch **34** is pressed onto the skin and the rest of the backing is removed to expose the rest of the adhesive lining that secures the patch **34** to the skin of the patient.

The bone probe needles **31** protective covering **32** is removed, and the bone probe needles **31** are placed on the target zone **37** with the axis of the apparatus **1** perpendicular to the skin of the patient. The hollow outer sleeve **5** is pushed into the target zone **37** until the release of the hollow outer sleeve **5** from the inner sleeve **6** is heard and felt. The hollow outer sleeve **5** is pulled straight back. The support sleeves **33** fall out leaving the infusion tube **17** with bone portal **21** embedded in the patient. A syringe is attached to the infusion tube **17** to withdraw marrow to verify that the infusion tube **17** is at the correct depth in the bone. The bone probe needles **31** protective covering **32** is put back on the apparatus **1** for safety reasons. The infusion tube **17** is connected to a connector tube **41** attached at the patch **34** through the tube connector **20** to provide slack in the tubing and less strain on the infusion site. The connector tube **41** is connected to a supply of intravenous drugs or fluid. The protective cover **44** is placed on the patch **34** so as to engage the covering fastening material **45** with the patch fastening material **38,** protecting the infusion tube **17** from dislodgment. After the infusion is complete, the infusion tube **17** may be removed by inserting a remover **23** into the infusion tube **17** and turning it clockwise to engage the threads in the bone portal **21** until the remover stops turning. The remover is then pulled straight out removing the infusion tube 17 from the patient.

As an example of the specifications of the apparatus 1 used to infuse the manubrium the following represent a possible design:

| | |
|---|---|
| Bone probe needles | Ten 1.27 mm hypodermic needles |
| | equi-spaced around the bone |
| | probe. |
| | angle φ = 15 degrees |
| | angle θ = 60 degrees |
| | Ball radius = 3.16 mm |
| | Maximum force on spring = 9.1 kg |
| | Activation distance of bone |
| | portal relative to end |
| | of bone probe needles = 8.87 mm |

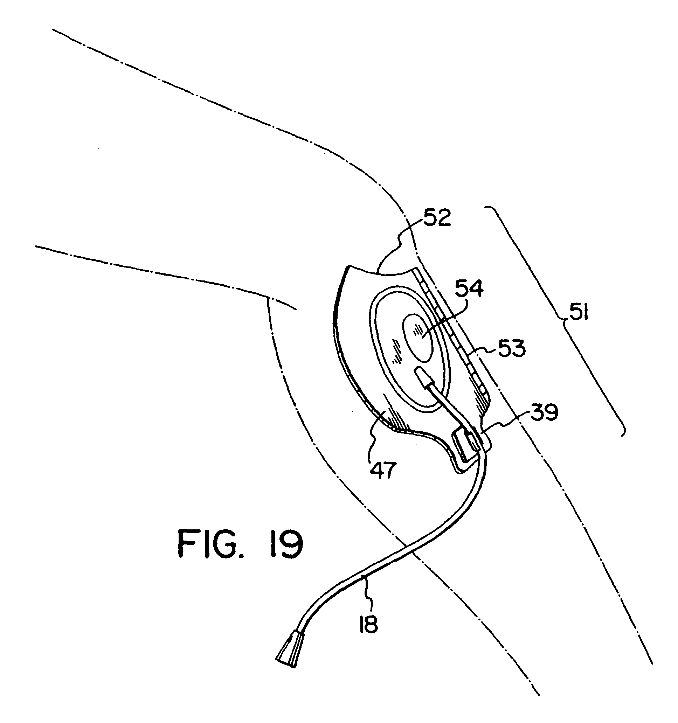

Referring to Fig. 19, a tibial target patch 51 designed for use at the tibial site for intraosseous infusion is shown. This is a site commonly used in children, and occasionaly used in adult patients. The tibial site target patch 51 has an alignment feature 52 that is aligned with the tibial tuberosity at the proximal end of the tibia. The tibial target patch 51 has a marking 53 on it for aligning with the ridge of bone that can be felt along the axis of the tibia. The tibial target patch 51 has an adhesive backing with a liner that is removed to place the patch on the skin. The tibial target patch 51 has a tibial target zone 54 that is used as a target for placing any intraosseous needle This invention removes the need for judging the distances from the anatomical landmarks. The patch could also have an instrument guide (not shown) that guides the needle into the bone at the recommended angle of 45 degrees.
The patch could also have loop fasteners for attaching a protective dome designed for placement at chis sice. The patch could also have a connector tubing bonded to the patch to remove stress and strain from the infusion tube or needle A similar target patch can easily be envisioned for use at other target sites, for example the distal end of the tibia, near the ankle; the distal end of the femur near the knee; the iliac crest site; or the distal end of the radius (lower arm).

## Claims

1. An apparatus for positioning an object a predetermined distance beneath a hard bone cortical layer of a patient covered by a layer of soft overlying tissue, having an operative end that is a bone penetrating end, and a remote end held by a user that is opposite to the bone penetrating end, a pusher (5, 16) coupled to said object (17), said pusher (5, 16) operative to receive force applied by the user and transfer said force to said object, a coupler (7) coupled to said pusher (5, 16) and operative to couple said pusher to said object during positioning of said object, a position probe (3) coupled to said object operative to penetrate said layer of soft overlying tissue and stop at said hard bone cortical layer and sense when said object reaches a predetermined depth below a surface of said bone cortical layer, a spring assembly (8) compressed between said position probe (3) and said pusher (5, 16) and a release mechanism (4, 7, 8, 9, 10, 29) operative to remove the force transferred to said object once said object reaches the predetermined depth.

2. Apparatus according to claim 1, wherein said pusher includes an outer cylindrical sleeve (5) having a ball race (9), and a cap (16), and an inner cylindrical sleeve (6) having a plurality of ball holes (10), slidably coupled within said outer cylinder sleeve (5) by said coupler (7).

3. Apparatus according to claim 2, wherein said coupler is a plurality of balls (7) positionable in said ball holes (10) in said inner cylindrical sleeve(6), and in said ball race (9) in said outer cylindrical sleeve so as to couple said inner and outer cylindrical sleeves together.

4. Apparatus according to claim 1, including a housing assembly (2), wherein said object is removably coupled to said housing (2), said release mechanism is a part of said housing assembly (2), and said pusher is a part of said housing assembly (2) to which a user applies force, and said release mechanism is operative to remove substantially all of the force applied to said object once a portion of said object has penetrated the predetermined depth in said hard shell of material.

5. Apparatus according to claim 4, wherein said hard shell of material is a bone cortical layer (40), said layer of soft material is skin and subcutaneous tissue (56, 57) overlying said bone cortical layer, and said housing assembly (2) is removably coupled to said object so that upon insertion into a patient, said object penetrates said bone cortical layer the predetermined distance and, upon withdrawal of said housing assembly (2), said object is left in the body of the patient.

6. Apparatus according to claim 5, wherein said object is an infusion tube (17) and said position probe (31) is a bone probe assembly (3) that includes a plurality of needles which abut and are stopped by said bone cortical layer, and a spring (13) that compresses and allows the infusion tube to advance beyond the position of the tips of said plurality of needles, said tips of said plurality of needles being positioned a predetermined distance from the position of said infusion tube within said bone cortical layer.

7. Apparatus according to claim 6, wherein said housing assembly (2) comprises:
(a) an outer sleeve (5) having a cylindrical shape and a ball race (9) in an interior surface thereof proximate to an open end thereof;
(b) an inner sleeve (6), having a cylindrical shape slidably insertable in said outer sleeve (5), and having a plurality of ball holes (10) circumferentially spaced about said inner sleeve (6);
(c) a plurality of balls (7) positionable to engage said ball race (9) of said outer sleeve (5) and to pass through corresponding ones of the ball holes (10) of said inner sleeve (6) thereby coupling said outer sleeve (5) to said inner sleeve (6);
(d) a spring assembly having a spring (13) situated between said bone probe assembly (3) and an end of said inner sleeve (6) remote from said bone probe assembly (3);
wherein said bone probe assembly (3) is slidable in said inner sleeve (6) and has an outer surface with a conical shape (29) that reduces in diameter towards an end of a part of the bone probe assembly that contacts the patient; and
wherein upon the application of force to said outer sleeve (5), said plurality of balls (7) couple said inner sleeve (6) to said outer sleeve (5) and cause said spring (13) to compress between said bone probe assembly (3) and an end of said inner sleeve (6) and said plurality of balls (7) to travel along said outer surface with a conical shape (29) of said bone probe assembly (3) to a reduced diameter of said bone probe until said infusion tube has penetrated said cortical layer (40) the predetermined depth and said outer sleeve (5) is released from said inner sleeve (6) by said balls (7) moving inward to the center until said outer sleeve (5) is no longer coupled to said inner sleeve (6).

8. Apparatus according to claim 7, including a coupling between said inner sleeve (6) and said bone probe assembly (3) which permits relative motion between said inner sleeve (6) and said bone probe assembly (3) to an extent slightly beyond that at which said release mechanism is activated.

9. Apparatus according to claim 8, wherein said coupling includes an elongated slot (11) in said inner sleeve (6) and a pin (28) engaging said slot in said bone probe assembly (3).

10. Apparatus according to claim 7, wherein said bone probe assembly (3) comprises a bone probe ring (4) having a conical surface portion (29) with a wider portion of said bone probe ring (4) at an end remote from said plurality of needles (31) and a narrower end adjacent to said plurality of needles (31), said plurality of needles (31) extending from said bone probe ring (4), said bone probe assembly (3) having a plurality of pins (28) protruding therefrom engageable with elongated pin slots (11) in said inner sleeve (6).

11. Apparatus according to claim 7, wherein said infusion (17) is for aspirating tissue from and infusing fluid into the bone marrow (55) and said infusion tube includes a bone portal (21) and a hollow flexible tube (18) affixed thereto.

12. Apparatus according tc claim 11, wherein said bone probe assembly (3) has an axial opening therethrough to permit passage of said infusion tube (17) and including two elongated support sleeves (33) for slidable insertion of said infusion tube (17) between said support sleeves (33) through the axial opening of said bone probe assembly (3) so as to support said infusion tube (17) during the application of force thereto.

13. Apparatus according to claim 11, wherein said bone probe assembly (3) comprises a plurality of bevel-tipped needles (31) which protrude from the bone probe ring (4) parallel to an axis thereof.

14. Apparatus according to claim 13, wherein said needles (31) encircle the bone portal (21).

15. Apparatus according to claim **13,** including a protective covering (32) which fits over said needles having a hard outer shell.

16. Apparatus according to claim **11,** wherein said housing assembly (2) includes a rigid stylet (50) passing through said hollow flexible tube (18) and contacting said bone portal (21) wherein force applied to said outer sleeve (5) translates to said inner sleeve (6), to said stylet (50) and to said bone portal (21).

17. Apparatus according to claim **11,** wherein said infusion tube (17) includes a tube connector (20) fastened to a distal end of said hollow flexible tube (18), said tube connector (20) connactable to an external connector tube to increase slack in said infusion tube (17), prevent stress on said infusion tube (17) and said bone portal (21) and facilitate the infusion of fluids into a patient.

18. Apparatus according to claim 7, wherein after movement of said balls (7) from said ball race (9), said spring (13) exerts a force on said bone probe assembly (3), causing the outer surface with a conical shape (29) around an exterior thereof to bias said balls outwardly against said outer sleeve (5) and functionally couple said bone probe assembly (3), said inner sleeve (6) and said outer sleeve (5) together.

19. Apparatus according to claim 16, wherein a bone penetrating end (62) of said bone portal (21) is tapered.

20. Apparatus according to claim 16, wherein said bone portal (21) is stainless steel.

21. Apparatus according to claim 16, wherein said bone portal (21) has a passageway along a length thereof open to a tip at one end and to an opposite end thereof.

22. Apparatus according to claim 16, including threads (22) formed in the passageway of said bone portal (21).

23. Apparatus according to claim 16, including a remover (23) wherein said remover (23) is an elongated rod having threads at one end which register with threads (22) in said passageway of said bone portal (21) so that upon application of an extraction force on said remover, said bone portal (21) is withdrawn from bone of a patient.

## Patentansprüche

1. Vorrichtung zum Positionieren eines Objekts in einem vorgegebenen Abstand unterhalb einer kortikaten Knochenhartschicht eines Patienten, die von einer Schicht eines weichen darüber liegenden Gewebes bedeckt ist, aufweisend ein Wirkende, welches ein in den Knochen eindringendes Ende darstellt, und ein fernes Ende, das von einem Benutzer gehalten wird und gegenüber dem in den Knochen eindringenden Ende liegt, einen Schieber (5, 16) der an das Objekt (17) gekoppelt ist, wobei der Schieber (5, 16), der dafür ausgelegt ist, eine Kraft aufzunehmen, die vom Benutzer aufgebracht wird, und die Kraft auf das Objekt zu übertragen, einen Koppler (7), der mit dem Schieber (5, 16) gekoppelt und dafür ausgelegt ist, den Schieber während des Positionierens des Objekts mit dem Objekt zu koppeln, einen Positionsfühler (3), der mit dem Objekt gekoppelt und dafür ausgelegt ist, die Schicht des weichen darüber liegenden Gewebes zu durchdringen und an der kortikalen Knochenhartschicht anzuhalten und zu erfassen, wann das Objekt eine vorgegebene Tiefe unterhalb der Oberfläche der kortikalen Knochenschicht erreicht, eine Federeinheit (8), die zwischen dem Positionsfühler (3) und dem Schieber (5, 16) komprimiert ist, und einen Freigabemechanismus (4, 7, 8, 9, 10, 29), der dafür ausgelegt ist, die auf das Objekt übertragene Kraft abzuziehen, sobald das Objekt die vorgegebene Tiefe erreicht hat.

2. Vorrichtung nach Anspruch 1, wobei der Schieber eine äußere zylindrische Hülse (5), welche ein Kugellager (9) aufweist, und eine Kappe (16) sowie eine innere zylindrische Hülse (6) mit einer Anzahl an Kugellöchern (10), welche verschiebbar innerhalb der äußeren zylindrischen Hülse (5) durch den Koppler (7) gekoppelt ist, umfasst.

3. Vorrichtung nach Anspruch 2, wobei der Koppler eine Anzahl an Kugeln (7) umfasst, welche in den Kugellöchern (10) in der inneren zylindrischen Hülse (6) positionierbar sind, sowie im Kugellager (9) in der äußeren zylindrischen Hülse, um die innere und die äußere zylindrische Hülse aneinander zu koppeln.

4. Vorrichtung nach Anspruch 1, aufweisend eine Gehäuseeinheit (2), wobei das Objekt entfernbar an das Gehäuse (2) gekoppelt ist, wobei der Freigabemechanismus ein Teil der Gehäuseeinheit (2) ist und der Schieber, auf welchen ein Benutzer eine Kraft ausübt, ein Teil der Gehäuseeinheit (2), und wobei der Freigabemechanismus dafür ausgelegt ist, im Wesentlichen die gesamte Kraft, die auf das Objekt ausgeübt wird, abzuziehen, sobald ein Teil des Objekts in die vorgegebene Tiefe in das Hartschalenmaterial eingedrungen ist.

5. Vorrichtung nach Anspruch 4, wobei das Hartschalenmaterial eine kortikale Knochenschicht (40) ist, die Schicht aus weichem Material Haut und subkutanes Gewebe (56, 57) ist, welche über der kortikalen Knochenschicht liegen, und die Gehäuseeinheit (2) entfernbar an das Objekt gekoppelt ist, so dass auf den Einschub in einen Patienten hin das Objekt die kortikale Knochenschicht um den vorgegebenen Abstand durchdringt und auf das Herausziehen der Gehäuseeinheit (2) hin das Objekt im Körper des Patienten gelassen wird.

6. Vorrichtung nach Anspruch 5, wobei das Objekt eine Infusionsröhre (17) ist und der Positionsfühler (31) eine Knochenfühlereinheit (30) ist, welche eine Anzahl an Nadeln umfasst, die an die kortikale Knochenschicht anstoßen und von dieser gestoppt werden, und eine Feder (13), welche komprimierbar ist und es der Infusionsröhre ermöglicht, über die Position der Spitzen der Anzahl an Nadeln hinaus vorzustoßen, wobei die Spitzen der Anzahl an Nadeln an einem vorgegebenen Abstand zur Position der Infusionsröhre innerhalb der kortikalen Knochenschicht positioniert werden.

7. Vorrichtung nach Anspruch 6, wobei die Gehäuseeinheit (2) folgendes aufweist:
(a) eine äußere Hülse (5) von zylindrischer Form und mit einem Kugellager (9) an einer Innenoberfläche derselben in der Nähe eines offenen Endes derselben;
(b) eine innere Hülse (6) von zylindrischer Form, welche gleitend in die äußere Hülse (5) einschiebbar ist und welche eine Anzahl an Kugellöchern (10) aufweist, die umfangsseitig um die innere Hülse (6) beabstandet sind;
(c) eine Anzahl an Kugeln (7) die so positionierbar sind, dass sie in das Kugellager (9) der äußeren Hülse (5) eingreifen und durch entsprechende der Kugellöcher (10) der inneren Hülse (6) laufen, wodurch die äußere Hülse (5) mit der inneren Hülse (6) gekoppelt wird;
(d) eine Federeinheit mit einer Feder (13), welche zwischen der Knochenfühlereinheit (3) und einem Ende der inneren Hülse (6) entfernt von der Knochenfühlereinheit (3) positioniert ist;
wobei die Knochenfühlereinheit (3) in der inneren Hülse (6) gleiten kann und eine äußere Oberfläche von konischer Form (29) aufweist, deren Durchmesser zum Ende des Teils der Knochenfühlereinheit hin, welches mit dem Patienten in Berührung kommt, abnimmt; und
wobei auf das Aufbringen einer Kraft auf die äußere Hülse (5) hin die Anzahl an Kugeln (7) die innere Hülse (6) an die äußere Hülse (5) koppelt und bewirkt, dass die Feder (13) zwischen der Knochenfühlereinheit (3) und einem Ende der innere Hülse (6) komprimiert wird und dass die Anzahl an Kugeln (7) entlang der äußeren Oberfläche mit konischer Form (29) der Knochenfühlereinheit (3) bis zu einem verringerten Durchmesser des Knochenfühlers vordringt, bis die Infusionsröhre die kortikale Schicht (40) über die vorgegebene Tiefe durchdrungen hat und die äußere Hülse (5) wird **dadurch**, dass die Kugeln (7) sich nach innen bis zum Zentrum bewegen, bis die äußere Hülse (5) nicht länger an die innere Hülse (6) gekoppelt ist, von der inneren Hülse (6) gelöst.

8. Vorrichtung nach Anspruch 7, umfassend eine Kupplung zwischen der innern Hülse (6) und der Knochenfühlereinheit (3), welche eine Relativbewegung zwischen der inneren Hülse (6) und der Knochenfühlereinheit (3) bis zu einer Ausdehnung zulässt, die geringfügig jenseits jener liegt, bei welcher der Freigabemechanismus aktiviert wird.

9. Vorrichtung nach Anspruch 8, wobei die Kupplung einen länglichen Schlitz (11) in der inneren Hülse (6) aufweist, sowie einen Stift (28), der mit dem Schlitz in der Knochenfühlereinheit (3) in Eingriff steht.

10. Vorrichtung nach Anspruch 7, wobei die Knochenfühlereinheit (3) einen Knochenfühlerring (4) aufweist, der einen konischen Oberflächenabschnitt (29) mit einem breiteren Abschnitt des Knochenfühlerrings (4) an dem Ende entfernt von der Anzahl an Nadeln (31) und ein schmäleres Ende benachbart der Anzahl an Nadeln (31) aufweist, wobei sich die Anzahl an Nadeln (31) von dem Knochenfühlerring (4) aus erstreckt, wobei die Knochenfühlereinheit (3) eine Anzahl an Stiften (28) aufweist, die aus dieser hervorstehen und mit länglichen Stiftschlitzen (11) in der inneren Hülse (6) in Eingriff bringbar sind.

11. Vorrichtung nach Anspruch 7, wobei die Infusionsröhre (17) dem Ansaugen von Gewebe aus dem Knochenmark (55) und dem Injizieren von Fluid in dasselbe dient und die Infusionsröhre ein Knochenportal (21) und eine hohle flexible Röhre (18), die daran befestigt ist, umfasst.

12. Vorrichtung nach Anspruch 11, wobei die Knochenfühlereinheit (3) eine axiale Durchgangsöffnung aufweist, um das Durchtreten der Infusionsröhre (17) zu ermöglichen, und zwei längliche Stützhülsen (33) für das gleitende Einführen der Infusionsröhre (17) zwischen die beiden Stützhülsen (33) durch die axiale Öffnung der Knochenfühlereinheit (3) zu ermöglichen, um die Infusionsröhre (17) während des Anlegens einer Kraft daran zu stützen.

13. Vorrichtung nach Anspruch 11, wobei die Knochenfühlereinheit (3) eine Anzahl an abgeschrägten Nadeln (31) aufweist, welche vom Knochenfühlerring (4) aus parallel zu dessen Achse vorstehen.

14. Vorrichtung nach Anspruch 13, wobei die Nadeln (31) das Knochenportal (21) umgeben.

15. Vorrichtung nach Anspruch 13, umfassend eine Schutzabdeckung (32), welche über die Nadeln passt und eine harte Außenschale aufweist.

16. Vorrichtung nach Anspruch 1, wobei die Gehäuseeinheit (2) einen starren Stiel (50) aufweist, der durch die hohle flexible Röhre (18) läuft und das Knochenportal (21) kontaktiert, wobei sich eine auf die äußere Hülse (5) ausgeübte Kraft auf die innere Hülse (6), auf den Stiel (50) und auf das Knochenportal (21) überträgt.

17. Vorrichtung nach Anspruch 11, wobei die Infusionsröhre (17) ein Röhrenverbindungsstück (20) aufweist, das an einem distalen Ende der hohlen flexiblen Röhre (18) befestigt ist, wobei das Röhrenverbindungsstück (20) an eine äußere Verbindungsröhre anschließbar ist, um die Lockerheit in der Infusionsröhre (17) zu erhöhen, Belastungen auf die Infusionsröhre (17) und das Knochenportal (21) zu verhindern und die Infusion von Fluiden in einen Patienten zu erleichtern.

18. Vorrichtung nach Anspruch 7, wobei nach der Bewegung der Kugeln (7) aus dem Kugellager (9) die Feder (13) eine Kraft auf die Knochenfühlereinheit (3) ausübt, wodurch bewirkt wird, dass die äußere Oberfläche mit konischer Form (29) um deren Außenseite herum die Kugeln nach außen gegen die äußere Hülse (5) vorspannt und die Knochenfühlereinheit (3) funktional mit der inneren Hülse (6) und der äußeren Hülse (5) verbunden wird.

19. Vorrichtung nach Anspruch 16, wobei das in den Knochen eindringende Ende (62) des Knochenportals (21) abgeschrägt ist.

20. Vorrichtung nach Anspruch 16, wobei das Knochenportal (21) aus Edelstahl ist.

21. Vorrichtung nach Anspruch 16, wobei das Knochenportal (21) entlang seiner Länge einen Durchgang aufweist, der für eine Spitze an einem Ende und für ein gegenüberliegendes Ende derselben offen ist.

22. Vorrichtung nach Anspruch 16, aufweisend Gewinde (22), die im Durchgang des Knochenportals (21) ausgebildet sind.

23. Vorrichtung nach Anspruch 16, aufweisend ein Entfernungsmittel (23), wobei das Entfernungsmittel (23) ein länglicher Stab ist, welcher Gewinde an einem Ende aufweist, welche mit Gewinden (22) im Durchgang des Knochenportals (21) zusammenwirken, so dass auf das Aufbringen einer Extraktionskraft auf das Entfernungsmittel das Knochenportal (21) aus dem Knochen eines Patienten gezogen wird.

## Revendications

1. Appareil pour positionner un objet à une distance prédéterminée en dessous d'une couche corticale osseuse dure d'un patient recouverte d'une couche de tissu mou reposant sur celle-ci, comportant une extrémité fonctionnelle qui est une extrémité pénétrant dans l'os ainsi qu'une extrémité éloignée tenue par un utilisateur qui est opposée à l'extrémité pénétrant dans l'os, un poussoir (5,16) couplé audit objet (17), ledit poussoir (5,16) fonctionnant pour recevoir une force appliquée par l'utilisateur et pour transférer ladite force audit objet, un coupleur (7) couplé audit poussoir (5,16) et fonctionnant pour coupler ledit poussoir audit objet pendant le positionnement dudit objet, une sonde de position (3) couplée audit objet fonctionnant pour pénétrer dans ladite couche de tissu mou situé au-dessus et pour s'arrêter à ladite couche corticale osseuse dure et pour détecter le moment où ledit objet atteint une profondeur prédéterminée en dessous d'une surface de ladite couche corticale osseuse, un ensemble formant ressort (8) comprimé entre ladite sonde de position (3) et ledit poussoir (5,16), ainsi qu'un mécanisme de relâchement (4, 7, 8, 9, 10, 29) fonctionnant pour supprimer la force transférée audit objet lorsque ledit objet atteint la profondeur prédéterminée.

2. Appareil selon la revendication 1, où ledit poussoir comprend un manchon cylindrique externe (5) ayant un chemin de roulement à billes (9) et un capuchon (16), et un manchon cylindrique interne (6) ayant plusieurs trous de billes (10) couplé d'une manière coulissante avec ledit manchon cylindrique externe (5) par ledit coupleur (7).

3. Appareil selon la revendication 2, où ledit coupleur est une pluralité de billes (7) pouvant être positionnées dans lesdits trous de billes (10) dans ledit manchon cylindrique interne (6), et dans ledit chemin de roulement à billes (9) dans ledit manchon cylindrique externe de manière à coupler lesdits manchons cylindriques interne et externe ensemble.

4. Appareil selon la revendication 1, comprenant un ensemble formant boîtier (2), où ledit objet est couplé amoviblement audit boîtier (2), ledit mécanisme de relâchement fait partie dudit ensemble formant boîtier (2), et ledit poussoir fait partie dudit ensemble formant boîtier (2) auquel un utilisateur applique une force, et ledit mécanisme de relâchement fonctionne pour supprimer sensiblement toutes les forces appliquées audit objet lorsqu'une portion dudit objet a pénétré selon la profondeur prédéterminée dans ladite coque de matériau dur.

5. Appareil selon la revendication 4, où ladite coque de matériau dur est une couche corticale osseuse (40), ladite couche de matériau mou est la peau et ledit tissu sous-cutané (56,57) reposant sur ladite couche corticale osseuse, et ledit ensemble formant boîtier (2) est couplé amoviblement audit objet de sorte que lors de l'insertion dans un patient, ledit objet pénètre dans ladite couche corticale osseuse selon la distance prédéterminée et, lors du retrait dudit ensemble formant boîtier (2), ledit objet reste dans le corps du patient.

6. Appareil selon la revendication 5, où ledit objet est un tube d'injection (17), et ladite sonde de position (31) est un ensemble de sonde osseuse (3) qui comprend plusieurs aiguilles qui butent contre et sont arrêtées par ladite couche corticale osseuse, et un ressort (13) qui comprime et permet au tube d'injection d'avancer au-delà de la position des pointes de ladite pluralité d'aiguilles, lesdites pointes de ladite pluralité d'aiguilles étant positionnées à une distance prédéterminée de la position dudit tube d'injection dans ladite couche corticale osseuse.

7. Appareil selon la revendication 6, où ledit ensemble de boîtier (2) comprend:
(a) un manchon externe (5) ayant une forme cylindrique et un chemin de roulement à billes (9) dans sa surface intérieure à proximité de son extrémité ouverte;
(b) un manchon intérieur (6) ayant une forme cylindrique pouvant être inséré d'une manière coulissante dans ledit manchon externe (5) et ayant plusieurs trous de billes (10) espacés circonférentiellement autour dudit manchon interne (6);
(c) une pluralité de billes (7) pouvant être positionnées pour s'engager dans ledit chemin de roulement à billes (9)dudit manchon externe (5) et pour passer à travers des trous de billes correspondants (10) dudit manchon interne (6) en couplant ainsi ledit manchon externe (5) audit manchon interne (6);
(d) un ensemble formant ressort comportant un ressort (13) situé entre ledit ensemble de sonde osseuse (3) et une extrémité dudit manchon interne (6) éloignée dudit ensemble de sonde osseuse (3);
où ledit ensemble de sonde osseuse (3) peut coulisser dans ledit manchon interne (6) et présente une surface extérieure d'une forme conique (29) dont le diamètre diminue vers une extrémité d'une partie de l'ensemble de sonde osseuse qui vient en contact avec le patient; et
où lors de l'application d'une force audit manchon externe (5), ladite pluralité de billes (7) relie ledit manchon interne (6) audit manchon externe (5) et amène ledit ressort (13) à être comprimé entre ledit ensemble de sonde osseuse (3) et une extrémité dudit manchon interne (6) et ladite pluralité de billes (7) à se déplacer le long de ladite surface extérieure d'une forme conique (29) dudit ensemble de sonde osseuse (3) à un diamètre réduit de ladite sonde osseuse jusqu'à ce que ledit tube d'injection ait pénétré à travers ladite couche corticale (40) selon la profondeur prédéterminée, et ledit manchon externe (5) est libéré dudit manchon interne (6) par lesdites billes (7) se déplaçant vers l'intérieur vers le centre jusqu'à ce que ledit manchon externe (5) ne soit plus couplé audit manchon interne (6).

8. Appareil selon la revendication 7, comprenant un couplage entre ledit manchon interne (6) et ledit ensemble de sonde osseuse (3) qui permet un mouvement relatif entre ledit manchon interne (6) et ledit ensemble de sonde osseuse (3) selon un degré légèrement au-delà de celui auquel ledit mécanisme de relâchement est activé.

9. Appareil selon la revendication 8, où ledit couplage comprend une fente oblongue (11) dans ledit manchon interne (6) et un axe (28) s'engageant dans ladite fente dans ledit ensemble de sonde osseuse (3).

10. Appareil selon la revendication 7, où ledit ensemble de sonde osseuse (3) comprend une bague de sonde osseuse (4) ayant une portion de surface conique (29) avec une portion plus large de ladite bague de sonde osseuse (4) à une extrémité éloignée de ladite pluralité d'aiguilles (31) et une extrémité plus étroite adjacente à ladite pluralité d'aiguilles (31), ladite pluralité d'aiguilles (31) s'étendant depuis ladite bague de sonde osseuse (4), ledit ensemble de sonde osseuse (3) ayant plusieurs axes (28) dépassant de celui-ci, aptes à s'engager dans les fentes d'axe oblongues (11) dans ledit manchon interne (6).

11. Appareil selon la revendication 7, où ledit tube d'injection (17) est destiné à aspirer le tissu de, et à infuser du fluide dans la moelle osseuse (55), et ledit tube d'injection comprend une porte osseuse (21) et un tube flexible creux (18) fixé à celle-ci.

12. Appareil selon la revendication 11, où ledit ensemble de sonde osseuse (3) présente une ouverture axiale à travers celui-ci pour permettre le passage dudit tube d'injection (17) et comprenant deux manchons de support oblongs (33) pour l'insertion coulissante dudit tube d'injection (17) entre lesdits manchons de support (33) à travers l'ouverture axiale dudit ensemble de sonde osseuse (3) de manière à supporter ledit tube d'injection (17) pendant l'application des forces à celui-ci.

13. Appareil selon la revendication 11, où ledit ensemble de sonde osseuse (3) comprend plusieurs aiguilles coupées en biais (31) qui font saillie de la bague de sonde osseuse (4) parallèle à un axe de celles-ci.

14. Appareil selon la revendication 13, où lesdites aiguilles (31) encerclent la porte osseuse (21).

15. Appareil selon la revendication 13, comprenant un recouvrement de protection (32) qui s'adapte sur lesdites aiguilles comportant une coque extérieure dure.

16. Appareil selon la revendication 11, où ledit ensemble de boîtier (2) comprend un stylet rigide (50) passant à travers ledit tube flexible creux (18) et venant en contact avec ladite porte osseuse (21) lorsque la force appliquée audit manchon externe (5) produit un mouvement de translation vers ledit manchon interne (6), audit stylet (50) et à ladite porte osseuse (21).

17. Appareil selon la revendication 11, où ledit tube d'injection (17) comprend un connecteur de tube (20) fixé à une extrémité distale dudit tube flexible creux (18), ledit connecteur de tube (20) pouvant être connecté à un tube de connecteur externe pour augmenter le relâchement dans ledit tube d'injection (17), en empêchant des contraintes sur ledit tube d'injection (17) et ladite porte osseuse (21) et pour faciliter l'injection de fluides dans un patient.

18. Appareil selon la revendication 7, où après le déplacement desdites billes (7) dudit chemin de roulement de billes (9), ledit ressort (13) exerce une force sur ledit ensemble de sonde osseuse (3) en amenant la surface extérieure d'une forme conique (29) autour d'un extérieur de celle-ci à solliciter lesdites billes vers l'extérieur contre ledit manchon externe (5) et à coupler fonctionnellement ledit ensemble de sonde osseuse (3), ledit manchon interne (6) et ledit manchon externe (5) ensemble.

19. Appareil selon la revendication 16, où une extrémité pénétrant dans l'os (62) de ladite porte osseuse (21) est effilée.

20. Appareil selon la revendication 16, où ladite porte osseuse (21) est de l'acier inoxydable.

21. Appareil selon la revendication 16, où ladite porte osseuse (21) présente un passage sur sa longueur ouvert à une pointe à une extrémité et à une extrémité opposée à celle-ci.

22. Appareil selon la revendication 16, comprenant des filets (22) formés dans le passage de ladite porte osseuse (21).

23. Appareil selon la revendication 16, incluant un élément de retrait (23), où ledit élément de retrait (23) est une tige oblongue avec des filets à une extrémité qui correspondent aux filets (22) dans ledit passage de ladite porte osseuse (21) de sorte que lors de l'application d'une force d'extraction audit élément de retrait, ladite porte osseuse (21) est retirée de l'os d'un patient.
